(19) **Europäisches Patentamt**

**European Patent Office**

**Office européen des brevets**

(11) **EP 0 932 388 B1**

(12) **EUROPEAN PATENT SPECIFICATION**

(45) Date of publication and mention
of the grant of the patent:
**29.03.2006 Bulletin 2006/13**

(51) Int Cl.:
**A61K 9/00** (2006.01)

(21) Application number: **97910713.3**

(22) Date of filing: **16.09.1997**

(86) International application number:
**PCT/US1997/016599**

(87) International publication number:
**WO 1998/014168 (09.04.1998 Gazette 1998/14)**

(54) **DOSAGE FORM AND METHOD FOR ADMINISTERING DRUG**

DOSIERUNGSFORM FÜR EINE VERZÖGERTE UND ZUNEHMENDE WIRKSTOFFSFREISETZUNG

FORME POSOLOGIQUE ET PROCEDE D'ADMINISTRATION DE MEDICAMENTS

(84) Designated Contracting States:
**AT BE CH DE DK ES FI FR GB GR IE IT LI LU NL PT SE**

(30) Priority: **30.09.1996 US 28726 P**
**12.11.1996 US 30514 P**
**22.04.1997 US 44121 P**

(43) Date of publication of application:
**04.08.1999 Bulletin 1999/31**

(73) Proprietor: **ALZA CORPORATION**
**Palo Alto**
**California 94303-0802 (US)**

(72) Inventors:
• **GUPTA, Suneel, K.**
**Sunnyvale, CA 94087 (US)**
• **GUINTA, Diane, R.**
**Palo Alto, CA 94303 (US)**
• **CHRISTOPHER, Carol, A.**
**Belmont, CA 94002 (US)**
• **SAKS, Samuel, R.**
**Burlingame, CA 94010 (US)**

(74) Representative: **Golding, Louise Ann**
**Frank B. Dehn & Co.,**
**179 Queen Victoria Street**
**London EC4V 4EL (GB)**

(56) References cited:
EP-A- 0 094 123          EP-A- 0 212 747
EP-A- 0 216 743          EP-A- 0 348 808
EP-A- 0 381 219          WO-A-93/05769
WO-A-98/06380            BE-A- 675 379
FR-A- 2 635 460          US-A- 2 738 303

• HUBBARD J. ET AL: J. PHARM. SC., vol. 78, no. 11, 1989, pages 944-947,

**Description**

## FIELD OF THE INVENTION

This invention pertains to both a novel dosage form and to a novel method for administering a drug for producing a therapeutic effect. The invention concerns more specifically a dosage form that administers at a sustained and continuously ascending rate a drug to produce a given therapy, and more specifically a method for producing a therapeutic effect by administering over a predetermined period at a sustained and continuously ascending rate a drug to produce a given therapy. The invention relates also to a dosage form and to a method for achieving a therapeutic effect by administering an initial dose of drug followed by a sustained and increasing dose of drug over an extended time.

## BACKGROUND OF THE INVENTION

For a long time, pharmacy and medicine in every society used drugs that produce effects on pain, mood, thought, feeling, behavior, and psychological personality. These drugs are represented by opioids, barbiturates, hypnotics, central nervous system stimulants, central nervous system depressants, psychostimulants, alcohols, cannabinoids, and catecholamines. In present medicine, one class of these drugs has become the standard invention for the management of Attention-Deficit Disorder, that is, the central nervous system stimulants.

The benefits perceived by parents, teachers, physicians, psychologists, social workers, and clinicians are dramatic for central nervous system drugs, and this has resulted in the widespread and acceptable use of central nervous system medication to treat Attention-Deficit Disorder. In 1994, the latest period for collecting data, it was observed that about two percent of the school-aged female population and about six percent of the school-aged male population, for a total of about two million patients, were administered the medication for Attention-Deficit Disorder.

Prior to the advent of this invention, the dosage form and the method for administering a drug, for example, a central nervous system acting drug, consisted in using a standard pharmaceutical dosage form. For example, one prior art dosage form and method for administering a drug, such as the central nervous system drug methylphenidate, consists in using an immediate release tablet containing the drug. This immediate release form delivers the drug by instant dumping of the drug and this produces uneven blood levels characterized by peaks and valleys. For an immediate release form containing methylphenidate which is characterized by a rapid onset and a short half-life to produce the intended therapy, multiple doses are needed each day that can result in swings in behavior and in attention as the medication loses its therapeutic effect. This dosage form does not provide the needed therapy over an extended time.

Another prior art dosage form for dispensing a drug is the sustained-release dosage form. A drug dispensed from a prior art sustained-release dosage form may ascend initially but not over the entire dosing interval, and it actually may decline over time. That is, these sustained-release dosage forms dispense a drug in a nonascending profile over time, as they do not provide a continuously increasing release rate per hour throughout the extended dosing period. This dosage form, additionally, may not provide the required duration of therapy and the appropriate blood pattern. For drugs that act on the central nervous system, like methylphenidate, dispensed from a sustained-release nonascending dosage form, the patient often develops an acute tolerance to the drug manifested by a shortened duration and a decrease in the intensity of the therapeutic effect needed for acceptable therapy. The prior sustained-release delivery is also devoid of means that compensate for its shortcomings inherent therein.

The above presentation teaches that a critical need exists for a novel dosage form and for a novel method for administering a drug that overcomes the shortcomings known to the prior art. This long-felt need exists for a dosage form and for a method for (1) administering the drug at a sustained-increasing rate that simultaneously reduces or eliminates the frequency of daily dosing; for (2) a dosage form and a method for administering the drug in a sustained-compensating dose to substantially compensate for acute tolerance to the drug thereby maintaining a preselected clinical response; for (3) a dosage form that administers the drug in a sustained-ascending profile clinically indicated for the management of Attention-Deficit Disorders; and, for (4) a dosage form and a method for administering the drug initially and in a sustained-ascending profile throughout the entire school day.

## OBJECTS OF THE INVENTION

Accordingly, in view of the above presentation, it is an immediate object of this invention to make available a dosage form that overcomes the shortcomings known in the prior art.

Another object of the invention is to provide a novel and unique dosage form that delivers a drug in a controlled increasing dose to a patient over time.

Another object of the invention is to provide a dosage form that administers a dose of drug to maintain the therapeutic effect of the drug in a patient that acquires tolerance to the drug by delivering the drug in a controlled-increasing dose over time to maintain the therapeutic effect, while concomitantly substantially avoiding the development of acute tolerance.

Another object of the invention is to make available a dosage form that delivers a dose of drug that essentially avoids or lessens the development of acquired tolerance in a patient administered a drug that leads to acute tolerance by the dosage form administering the drug in a sustained and increasing dose over time.

Another object of the invention is to provide a dosage form for administering a central nervous system drug that overcomes the shortcomings known to the prior art.

Another object of the invention is to provide an improvement in a dosage form, wherein the improvement comprises the dosage form administering the drug in a sustained and constantly ascending profile over time for treating Attention-Deficit Disorder.

Another object of the invention is to provide a pharmacological composition as a solid dosage form comprising 1 mg to 500 mg of drug in admixture with a pharmaceutically acceptable carrier that is released in a sustained release and increasing dose for use in the treatment of psychological personalities.

Another object of the invention is to provide a dosage form for increasing the administration of a central nervous system acting drug per hour throughout a school day of 4 to 8-1/2 hours.

Another object of the invention is to provide a dosage form for administering a drug possessing central nervous system therapy in a sustained-increasing rate and at a minimum dose per day.

Another object of the invention is to provide a dosage form for administering a central nervous system acting drug in a drug delivery pattern that compensates for acquired tolerance associated with the drug.

Another object of the invention is to provide a dosage form that administers a drug for treating Attention-Deficit Disorder that comprises administering orally to a human diagnosed as having the disorder the drug in a sustained and increasing dose of 100 ng to 375 mg over 16 hours for treating the disorder in a human.

Another object of this invention is to provide a novel and unique method for maintaining the therapeutic effect of a drug in a patient that acquires tolerance to the drug, wherein the method comprises administering a dosage form to the patient that delivers the drug in a controlled increasing dose over an extended time to maintain the therapeutic effect, while concomitantly substantially avoiding the development of acute tolerance in the patient.

Another object of the invention is to make available a method for essentially avoiding or lessening the development of the acquired tolerance in a patient administered a drug that develops acute tolerance in the patient, wherein the method comprises administering the drug in a sustained and increasing dose over time to produce the intended effect.

Another object of the invention is to provide an improvement in a method for administering a drug, wherein the improvement comprises administering the drug in a sustained and constantly ascending profile over an extended time for treating Attention-Deficit Disorder.

Another object of the invention is to provide a method for administering a central nervous system acting drug in a continuously increasing release rate per hour throughout a school day of 4 to 8-1/2 hours.

Another object of the invention is to provide a novel method to compensate for acute tolerance development associated with a drug possessing the ability to produce tolerance in a patient, by administering the drug orally in a sustained-ascending dose to substantially lessen the unwanted effects of acute tolerance.

Another object of the invention is to provide an improvement in a method for administering a drug possessing central nervous system stimulant therapy, wherein the improvement comprises administering the drug in a sustained and ascending pattern over an extended time for treating Attention-Deficit Disorders and additionally provides therapeutic compensation for acquired tolerance associated with the drug.

Another object of the invention is to provide a method for treating Attention-Deficit Disorder comprising administering orally to a human diagnosed as having the disorder at a sustained and increasing dose of 100 ng to 500 mg over 16 hours a central nervous system drug for treating the disorder in a human patient and at a minimum number of doses per day.

Another object of the invention is to administer a central nervous system acting drug by a method wherein the drug ascends initially and continuously over the entire dosing interval for treating Attention-Deficit Disorder, ADD, and Attention-Deficit Hyperactivity Disorder, ADHD.

These objects, as well as other objects, features, and advantages of the invention will become more apparent from the following detailed disclosure of the invention and the accompanying claims.

## DRAWING FIGURES PROVIDED BY THE INVENTION

In Figure 1, the solid line depicts the plasma concentration for a central nervous system stimulant administered from an immediate release form, and the dotted line denotes the plasma concentration for a central nervous system stimulant released from a sustained nonascending form.

In Figure 2, an immediate release dosage form is depicted by the solid line comprising a peak and a valley depicted against a sustained release ascending dose shown by the dotted line.

In Figures 3, 4, and 5, the release results are depicted for a central nervous system drug wherein the clear circles denote a placebo, the dark circles denote an immediate release form, the dark squares denote a sustained-nonascending release profile, and clear squares denote a sustained ascending release rate profile.

Figures 6-8 depict the plasma methylphenidate concentration obtained by administering the drug three times a day, in an ascending dose, and by a dosage form that controls the delivery profile.

Figures 9-13 depict the plasma methylphenidate concentration obtained by administering the drug from a dosage form comprising an external overcoat initial dose of drug followed by an internal ascending dose of drug over time.

## DETAILED DISCLOSURE OF SPECIFICATION

In accordance with the practice of this invention, it has now been found that a dosage form and a method can be provided that administers a drug selected from methylphenidate, amphetamine, dextroamphetamine, methamphetamine, phenylisopropylamine, pemoline, and pharmaceutically acceptable salts thereof in a novel program that substantially lessens or completely compensates for tolerance in a patient. Tolerance, as defined in Pharmacology in Medicine, by Brill, p. 227 (1965) McGraw-Hill, is characterized as a decrease in effect followed by administering a drug. When tolerance develops following a single dose or a few doses over a very short time, it is referred to as acute tolerance. When the drug is administered over a more protracted period of time to show a demonstrable degree of tolerance, it is referred to as chronic tolerance. The medical literature, as exemplified in, The Pharmacological Bases of Therapeutics, by Goodman and Gilman, 8th Ed., p. 72 (1990) Pergamon Press, reported tolerance may be acquired to the effects of many drugs and this literature classifies tolerance as acute or chronic based on when it is acquired. That is, acute tolerance develops during a dosing phase of one dose or on one day, and chronic tolerance is acquired due to chronic administration typically weeks, months, and years. Tolerance as presented in medical literature most frequently denotes chronic tolerance as seen by administering larger doses over a long time, often necessitated by an increase in body dimensions, hepatic enzyme involved in biotransformation, and the like.

The invention comprises dosage forms for providing an ascending dose of drug selected from methylphenidate, amphetamine, dextroamphetamine, methamphetamine, phenylisopropylamine, pemoline, and pharmaceutically acceptable salts thereof. The dosage form of the invention comprises drug releasing beads and is capable of delivering the drug in a continuously increasing release rate per hour for about 4 to 8½ hours. The drug releasing beads are characterized by a dissolution profile wherein 0 to 20% of the beads undergo dissolution and release the drug in 0 to 2 hours, 20 to 40% undergo dissolution and release the drug in 2 to 4 hours, 40 to 60% exhibit dissolution and release in 4 to 6 hours, 60 to 80% in 6 to 8 hours, and 80 to 100% in 8 to 10 hours. The drug releasing beads comprise a central composition or core comprising a drug and pharmaceutically acceptable composition forming ingredients including a lubricant, antioxidant, and buffer. The beads comprise increasing doses of drug, for example, 1 mg, 2 mg, 5 mg, and 10 mg, increasing to 40 mg. The beads are coated with a release rate controlling polymer that can be selected utilizing the dissolution profile disclosed above. The manufacture of beads is disclosed in Inter. J. of Pharm., by Liu, Vol. 112, pp. 105-116 (1994); Inter. J. of Pharm., by Liu and Yu, Vol. 112, pp. 117-124 (1994); Pharm. Sci., by Remington, 14th Ed. pp. 1626-1628 (1970); J. Pharm. Sci., by Fincher, Vol. 57, pp. 1825-1835 (1968); and U.S. Patent No. 4,083,949.

The disclosed dosage forms of the invention may be used for administering a drug as hereinbefore described to a patient that may acquire acute or chronic tolerance for decreasing and/or avoiding said tolerance, and presently the dosage form is indicated for treating patients that may acquire acute tolerance. Further, in accordance with the practice of this invention, in one embodiment, it has also been found a dosage form that administers a drug for treating Attention-Deficit Disorder, to a human orally as a function of time to achieve the desired drug concentration over time. The concentration of drug relates to the dose of drug in mg per hour delivered per unit time in hours for absorption into the systemic circulation. The dosage form of the invention uniquely provides a method for maintaining a desired drug effect by adjusting continually the drug delivery rate when the therapeutic effect declines during acquired acute tolerance.

It is standard medical practice, that a drug should provide a therapeutic effect throughout the dosing interval. However, when tolerance develops or is acquired to a drug, the prior art approach to ensure a therapeutic response is to increase the dose administered in an immediate dose dumping manner, and for this type of dosing, where associated side effects are likely to occur, tolerance may develop unequally to all the effects, and the therapeutic index may decrease. Another approach used by the prior art to lessen the occurrence of tolerance is to administer drug doses less frequently so that acquired tolerance is avoided, but with this approach there is an absence of therapy for a given time.

In medicine, it is generally accepted that central nervous system acting drugs are useful for the management of Attention-Deficit Disorders. The drugs useful for this therapy are the mild central nervous system stimulants, and they include catecholamines and drugs that can mimic their action. The drugs used in the dosage form of the invention comprise a member selected from the group consisting of amphetamine, dextroamphetamine, methamphetamine, methylphenidate, racemic methylphenidate, threo-methylphenidate, phenylisopropylamine and pemoline. The drugs include also their pharmaceutically acceptable salts such as a member selected from the group consisting of hydrochloride, sulfate, phosphate, acetate, hydrobromide, pamoate, and maleate. A patient receiving these drugs typically acquires tolerance to the effects of the drugs. For example, a patient on methylphenidate and receiving a 5 mg dose twice a day acquires tolerance, and a larger dose must be administered due to the single large dose needed to overcome the tolerance development which would give rise to unwanted side effects. In some patients, the therapeutic response to methylphenidate declines in 4 to 5 hours despite the maintenance of methylphenidate in a nonascending constant concentration. That is, tolerance is acquired to the behavioral and psychological effects of methylphenidate and generally to psychostimulants. This invention has found also that a sustained release product that dispenses a noncompensating but constant concentration of a drug will not be clinically effective, as a sustained-release dosage form designed to produce a constant plasma of, for example, methylphenidate concentration, lacks efficacy particularly against acquired tolerance.

This invention among its objects provides a dosage form for treating Attention-Deficit Disorders, which include Attention-Deficit/Hyperactivity Disorder, combined type, predominantly inattentive type, predominantly hyperactive impulsive type and which are known also as minimal brain dysfunction, hyperkinetic child syndrome, behavioral syndrome, minimal cerebral dysfunction and minor cerebral dysfunction, as disclosed in the Diagnostic and Statistical Manual of Mental Disorders, 3rd Edition, pp. 49-56 (1987) published by the American Psychiatric Association, Washington, D.C., by making available a dosage form that substantially negates the unwanted results of the prior art by providing continuous compensation of drug to essentially eliminate acute tolerance, thereby producing a stabilization of the therapeutic effect.

The pharmacological effect of a drug is related to its receptor site concentration. Thus, when the effect of a drug is considered as a function (f) of delivery time (t), the kinetics of stabilization for some drugs can be rapid, and for other drugs the effect does not stabilize as a diminution in response expressed as tolerance develops to the drug. This latter condition applies to central nervous system acting drugs such as methylphenidate. This invention compensates for acquired-acute tolerance by providing an optimal drug delivery profile for its management. For some drugs the onset of tolerance is quick, for instance, tolerance develops for methylphenidate within hours after its administration. A management program for this is provided by this invention by making available a drug delivery pattern that initially delivers a dose of drug to achieve instant therapy and accompanied by a sustained-ascending release dose over time to maintain the effect.

The drug methylphenidate is commercially available in a sustained release dosage form Ritalin®-SR, and the methylphenidate dispensed by this commercial form can lead to acute tolerance. When acute tolerance is acquired a drug-free washout period of several hours is needed before a repeat administration is likely with the dosage form. The present invention however, compensates for the loss of a therapeutic effect of a drug, such as methylphenidate, by providing a method of delivery rate in mg per hour that continually compensates for the development of acute tolerance, by considering the clinical effect (E) of a drug at time (t) as a function of the drug concentration (C) according to Equation 1:

$$\text{Effect} = f(t, C) \qquad\qquad \text{Eq. 1}$$

In addition, the rate of drug delivered (A), in mg per hour is directly proportional to the concentration times the clearance of the drug. As the effect varies with time and the functionality is expressed, then according to this invention (A) can be governed to ensure the therapeutic effect is maintained at a clinical value. If the effect from a drug is found clinically to decrease with time, this decline could be linear as expressed by Equation 2:

$$\text{Effect}_{(t)} = \text{Effect}_{(ini)} - k_{Effect}*t \qquad\qquad \text{Eq. 2}$$

wherein, Effect $_{(ini)}$ is the clinical effect observed initially at the start of drug administration and Effect $_{(t)}$ is the effect observed at time (t) hours, $k_{effect}$ is a proportionality constant ascertained by measuring the clinical effect (E1) at time (t1) hours and (E2) at time (t2) hours while maintaining a constant plasma concentration followed by dividing (E1) minus (E2) by (t1) minus (t2). In order to maintain a constant effect, (A) must be adjusted with the same functionality according to Equation 3:

$$A_{(t)} = A_{(ini)} + k_{Effect}*t \qquad\qquad \text{Eq. 3}$$

wherein $A_{(ini)}$ is the initial drug input in mg per hour at the start of the therapy and $A_{(t)}$ is the drug input at time (t) hours, and $k_{Effect}$ is the proportionality constant presented above. If the therapeutic effect is found to decline exponentially with time, this relationship is expressed by Equation 4:

$$\text{Effect}_{(t)} = \text{Effect}_{(ini)} * \exp^{(-k\text{Effect}*t)} \qquad \text{Eq. 4}$$

wherein $\text{Effect}_{(ini)}$ and $\text{Effect}_{(t)}$ are as defined before, $k_{Effect}$ is a rate constant ($h^{-1}$), a unit of reciprocal hours, ascertained by measuring the clinical effect (E1) at time (t1) hours and (E2) at time (t2) hours while maintaining a constant plasma concentration followed by dividing natural log of (E1) minus natural log of (E2) by (t1) minus (t2). To maintain a constant effect, (A) must be adjusted according to Equation 5:

$$A_{(t)} = A_{(ini)} * \exp^{(k\text{Effect}*t)} \qquad \text{Eq. 5}$$

wherein $A_{(ini)}$ and $A_{(t)}$ is as defined before. $k_{Effect}$ is the rate constant ($h^{-1}$) presented above. The equations are presented in Pharmac. Ther., Vol. 16, pp. 143-166 (1982) by Holford N.H.G. and Sheiner, L.E.

The effects defined herein refer to the pharmacological effects exhibited by the drug as ascertained by clinical subjective observation such as SKAMP and CLAM, or as ascertained by objective activity monitoring such as mathematic tests and school accomplishments. The CLAM Test is a behavior rating that indicates social conformity or rebellion as developed by Conners, Lonez, and Milch and SKAMP is a rating that also measures behavior as developed by Swanson and reported in Psychopharmacological Bulletin, Vol. 21, pp. 887-890 (1985).

The effect measurements in this study were: (1) observer ratings on SKAMP scale (during classroom time) and (2) performance on the computerized mathematics test. Each child was tested on the mathematics test before the study began, and based on this pre-test during the study, a mathematics test appropriate for each child's ability was given. The morning and evening parent CLAM assessments were used to identify unusual behaviors. The evening parent CLAM was used to determine the presence of treatment effects in the evening hours, particularly treatment effects on the time the child fell asleep, and whether the child's sleep was interrupted. All of the children also wore an activity monitor (Actigraph) which records the movements of the child throughout the day. The activity (number of movements per minute) is recorded electronically and modeled as a function of the drug effect.

## DETAILED DISCLOSURE OF EXAMPLES PROVIDED BY THE INVENTION

The following examples are merely illustrative of the present invention.

## EXAMPLE 1

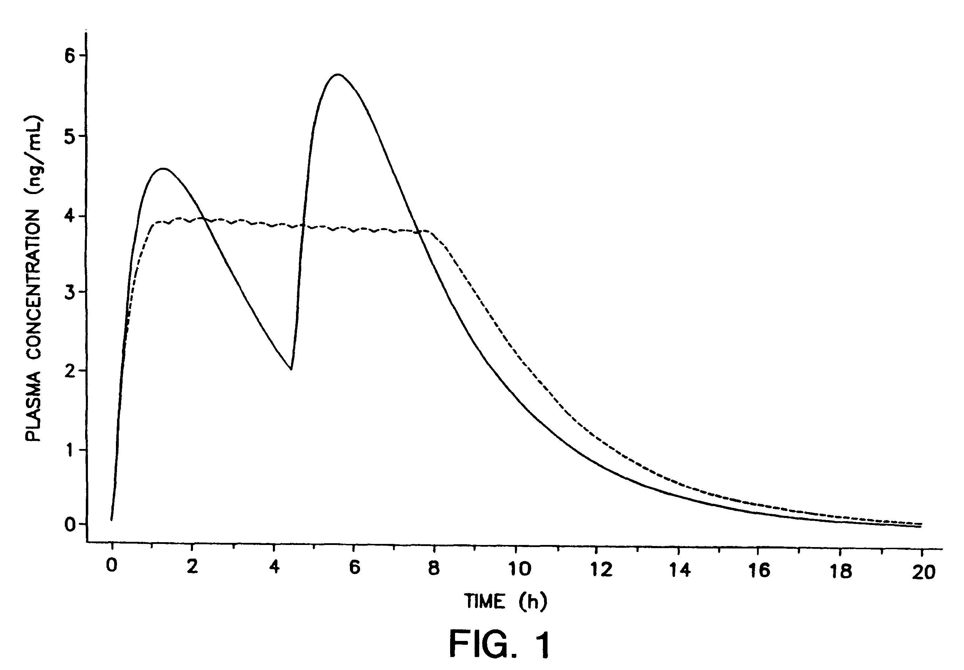

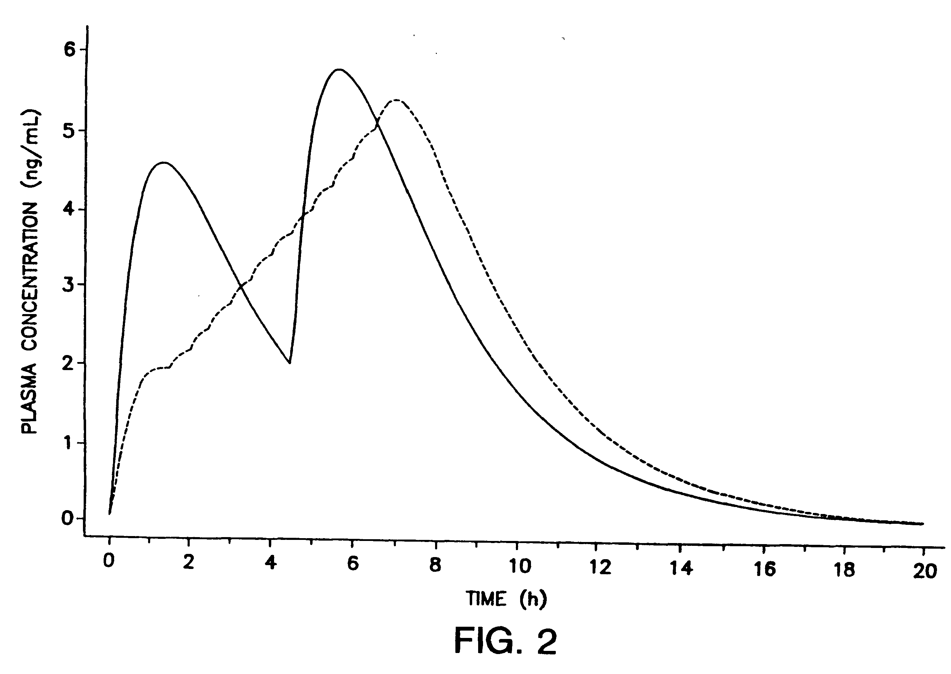

A commercially available immediate release tablet consisting of 5 mg of methylphenidate was administered twice a day to 36 school children, and the predicted plasma concentration in ng/ml graphed against time as seen in the solid black line in Figure 1. The tablet exhibits a peak and valley plasma concentration for the methylphenidate. A sustained-release nonascending program that administered 20 mg of methylphenidate consisting of 8.3 mg at zero hour followed by 0.9 mg at 1.5 hours, 0.9 mg at 2 hours, 0.9 mg 2.5 hours, 0.9 mg at 3 hours, 0.9 mg at 3.5 hours, 0.9 mg at 4 hours, 0.9 mg at 4.5 hours, 0.9 mg at 5 hours, 0.9 mg at 5.5 hours, 0.9 mg at 6 hours, 0.9 mg at 6.5 hours, 0.9 mg at 7 hours, and 0.9 mg at 7.5 hours produced the sustained-release dotted line parallel to the x-axis as seen in Figure 1. The immediate release tablet and the sustained-release dosage form were compared to a sustained-release dosage form that administered methylphenidate in an ascending profile. The sustained-release ascending profile corresponds to administering 4.2 mg at zero hour, 1.1 mg at 1.5 hours, 1.1 mg at 2 hours, 1.2 mg at 2.5 hours, 1.2 mg at 3.0 hours, 1.3 mg at 3.5 hours, 1.3 mg at 4 hours, 1.5 mg at 4.5 hours, 1.5 mg at 5 hours, 1.8 mg at 5.5 hours, 1.8 mg at 6 hours, and 2.0 mg at 6.5 hours, to produce the sustained-ascending release dotted line profile seen in Figure 2.

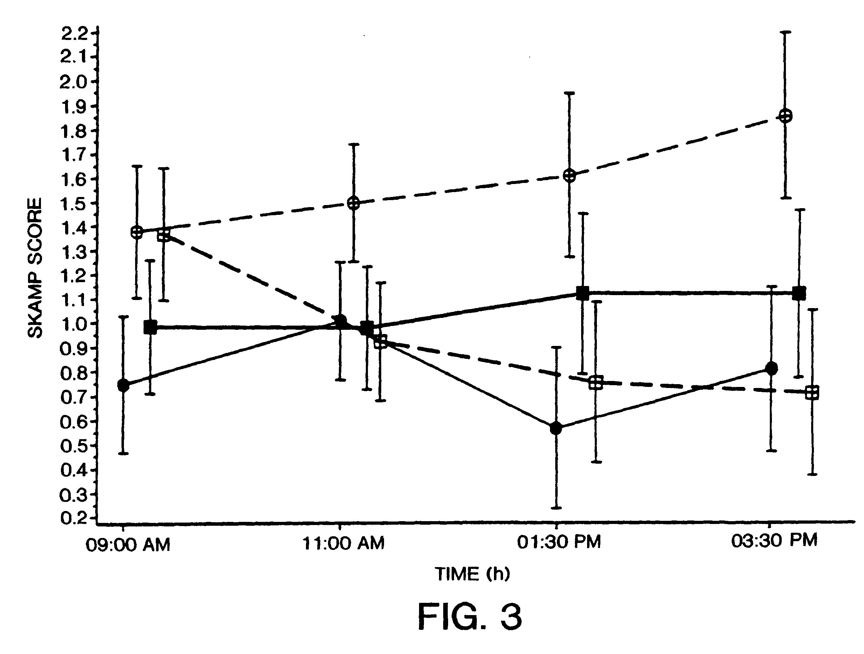

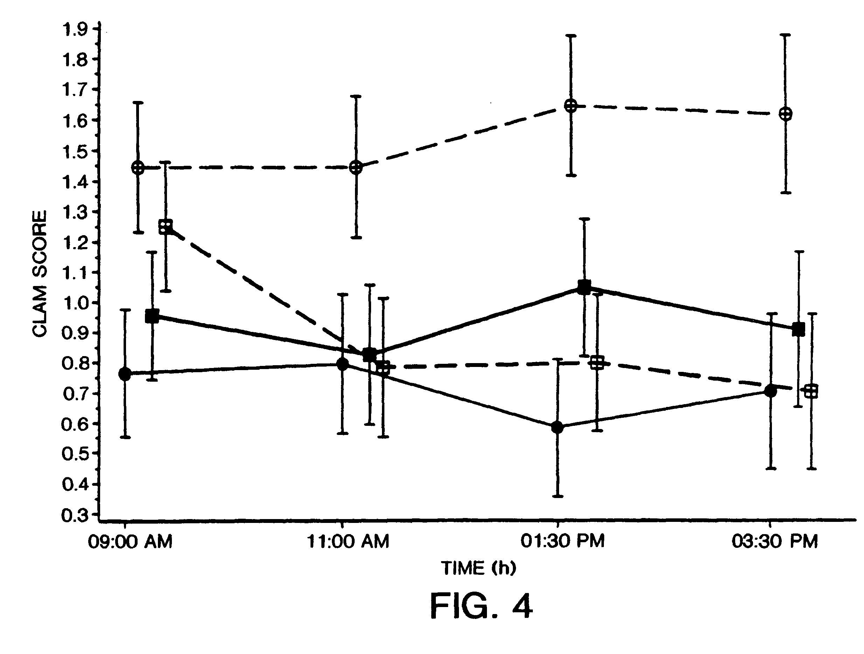

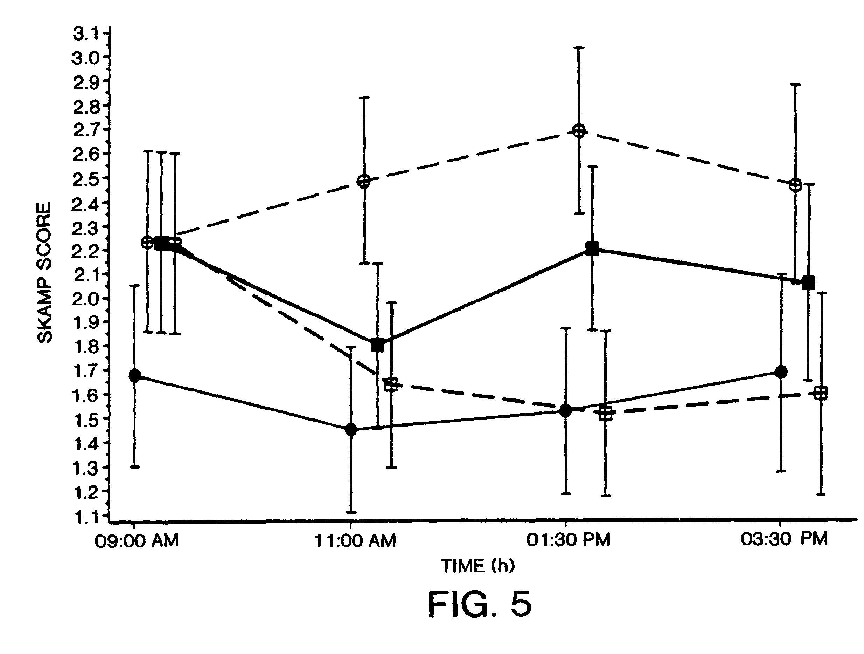

The results of the clinical studies demonstrated patients administered a dosage form free of methylphenidate, a placebo, exhibited high, elevated swings in behavior, such as activity, inappropriate behavior, low attention, lower mathematical scores and a disinterest in school. The patients administered a sustained-nonascending dose of methylphenidate exhibited a decrease in activity, higher mathematical scores and a lessening of inappropriate behavior. However, these effects were accompanied by the development of acute tolerance in the patient. The patient administered methylphenidate, according to this invention, in a controlled-sustained ascending profile exhibited the desired therapeutic effect without tolerance. The accompanying figures present the results of the above-described study. In Figures 3, 4, and 5, the line with a clear circle denotes a placebo, the dark circle an immediate release dosage form administered twice a day, the dark squares a sustained release nonascending dosage profile, and the clear squares the sustained ascending release profile provided by this invention. The SKAMP Score and CLAM Score were defined earlier in the specification, and the times are as indicated on the figures. Figure 3 denotes the observed behavior, Figure 4 denotes the inattention overactivity, and Figure 5 denotes the combined attention results of the study.

## EXAMPLE 2

A method for administering a drug in a sustained-increasing release rate is provided by administering a dosage form manufactured as a pharmaceutically acceptable gelatin two-piece joined capsule comprising a multiplicity of spherical beads. The capsule comprises a series of beads consisting of a progression of 1, 1.25, 1.5, 1.75, 2 and 2.25 mg of drug in each different bead coated correspondingly with a progression of 0.5, 1, 1.5, 2.5, 3, and 3.25 mm of polymeric poly(2.2-dioxo-trans-1, 4-cyclohexane dimethylene tetrahydrofuran). As the beads erode in the environment of the gastrointestinal tract they dispense drug in a sustained-ascending release rate over time. The drugs that can be dispensed by this method comprise a member selected from the group consisting of amphetamine, dextroamphetamine, methamphetamine, methylphenidate, phenylisopropylamine and pemoline. Procedures for coating are disclosed in J. Am. Phar. Assoc., Sci. Ed., Vol. 48, pp. 451-454 (1959); and U.S. Patent No. 2,799,241.

## EXAMPLE 3

A delivery system is provided by the invention which releases the drug resulting in an ascending plasma methylphenidate concentration time profile that substantially overcomes tolerance and maintains the desired pharmacological effect of the stimulant methylphenidate for the desired duration. For example, to achieve an effect-time profile similar to the three doses of immediate release given every 4 hours for 12 hours every day, TID (three times a day), a delivery system which results in the plasma methylphenidate concentrations between the ranges as listed below will overcome tolerance and maintain pharmacological effects. To make a delivery system which is equal to two doses of immediate release given every 4 hours the release rate can be truncated, and similarly, for the longer duration the concentration can be increased. The delivery profile exemplifies the drug and pharmacological effect. However, the concept of increasing concentration still remains the same.

Table 1 provides this range as a fraction of the simulated TID concentrations. The attached figures show the ascending profile variations superimposed on the TID and the reference ascending (ASCEND) treatment profiles.

| Time (h) | TID Concentration (ng/MI) | Ascending Profile Range (Fraction of TID Concentration) | |
|---|---|---|---|
| | | Low High | |
| 1.5 | 4.8 (peak) | 0.75 | 0.90 |
| 3.0 | 3.8 | 1.07 | 1.37 |
| 4.0 | 2.8(trough) | 1.32 2.29 | |
| 5.5 | 6.5(peak) | 0.80 | 1.20 |
| 7.0 | 4.8 | 1.42 | 1.81 |
| 8.0 | 3.6(trough) | 2.17 | 2.50 |
| 9.5 | 7.0(peak) | 1.10 | 1.23 |
| 11.0 | 5.2 | 1.00 | 1.38 |
| 12.0 | 3.9 | 0.97 | 1.54 |
| 15.0 | 1.7 | 1.00 | 1.94 |

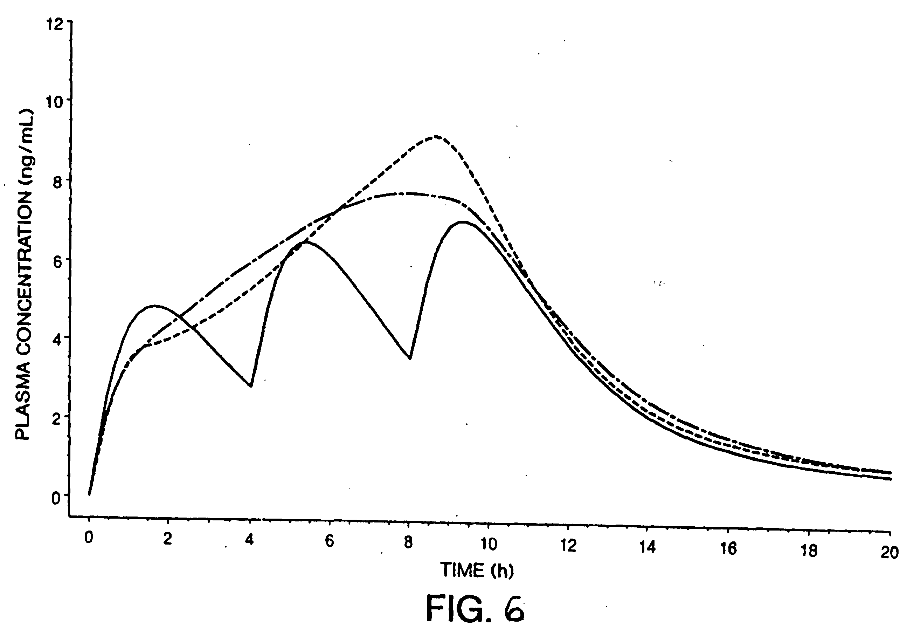

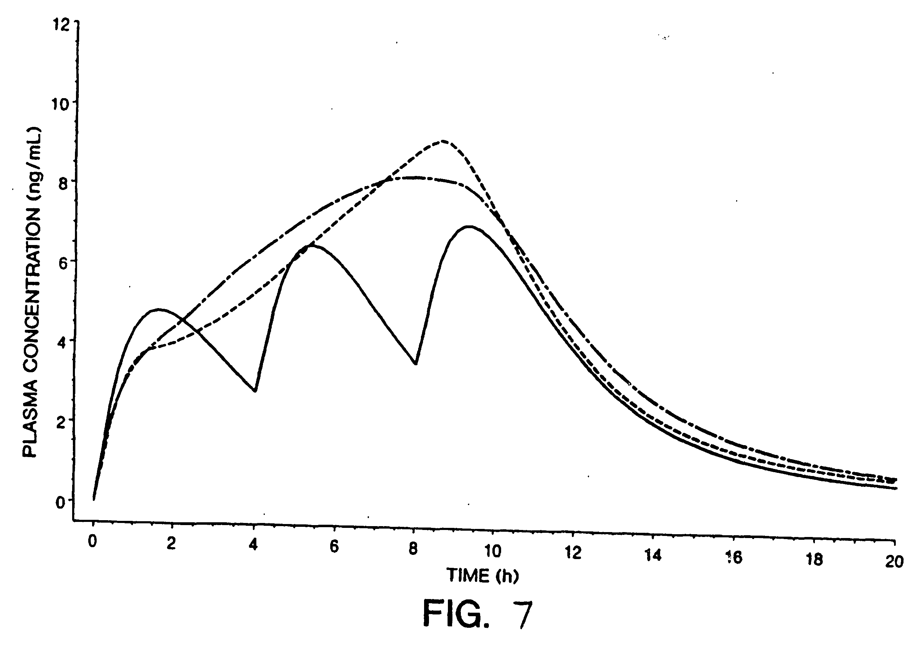

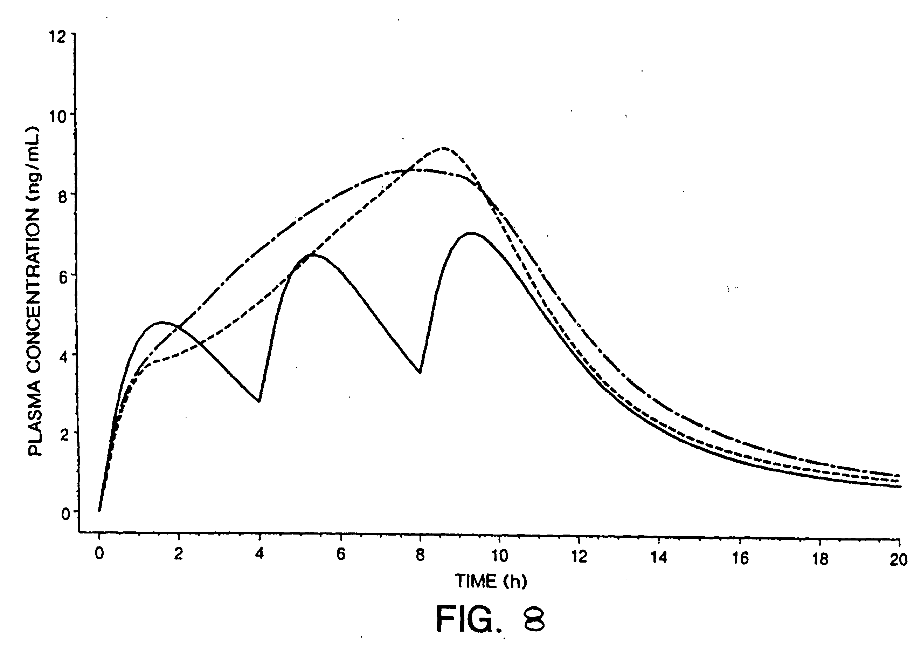

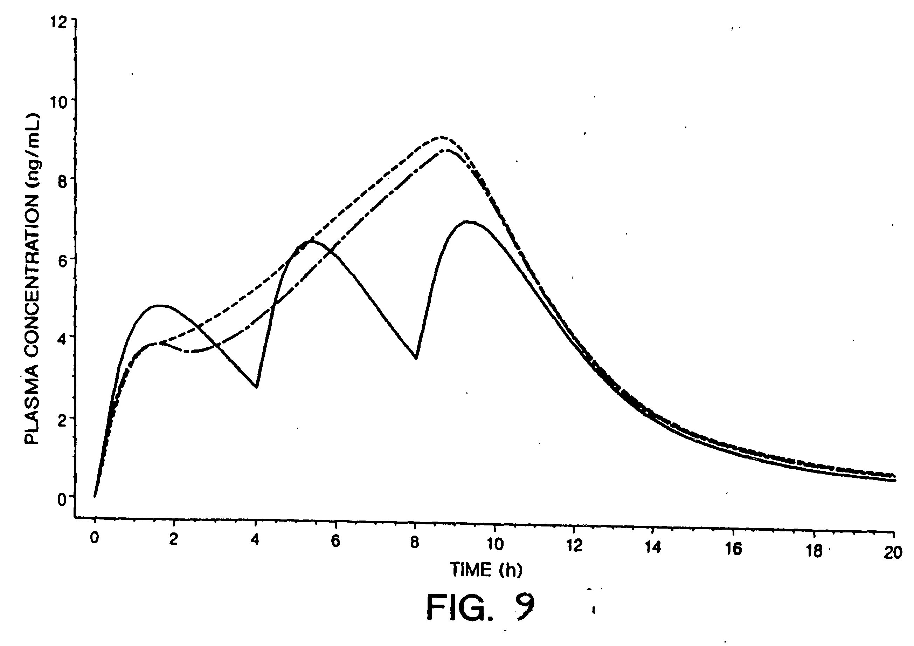

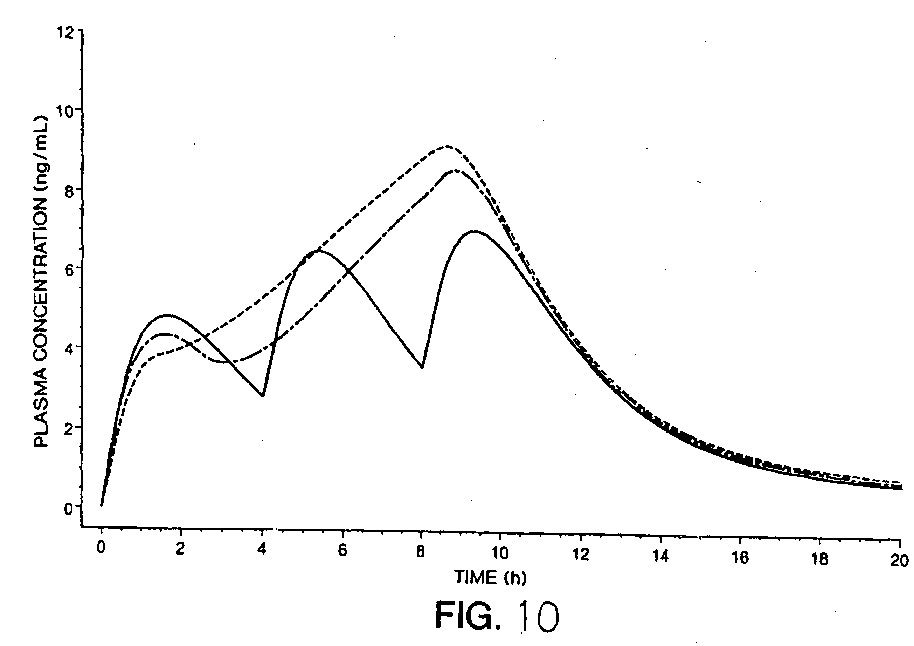

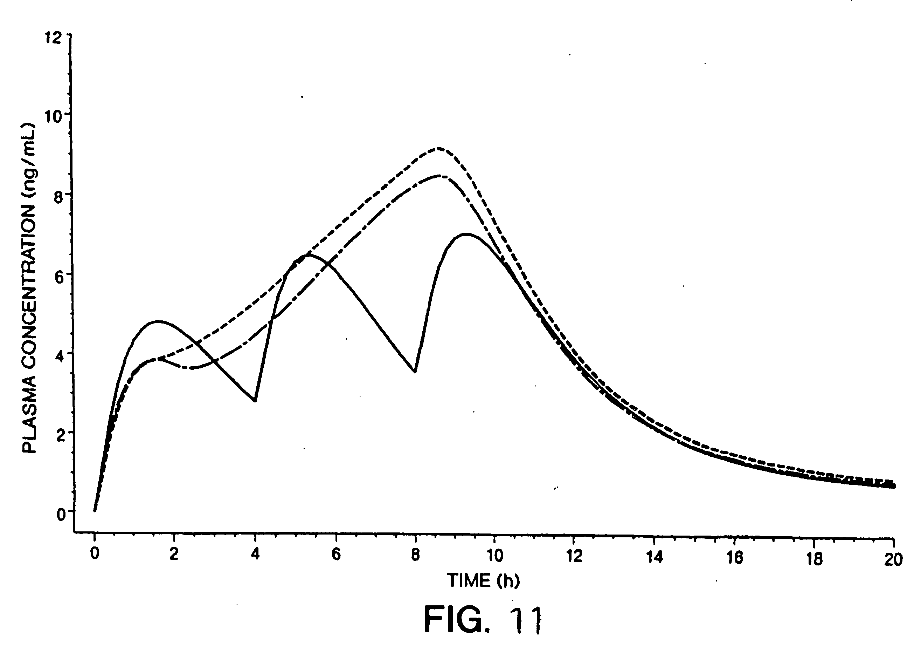

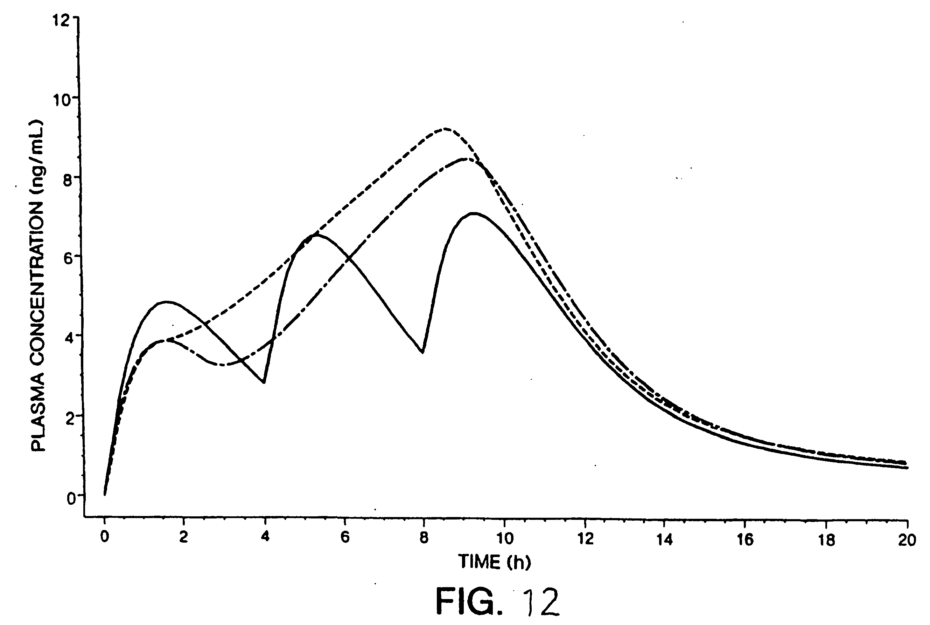

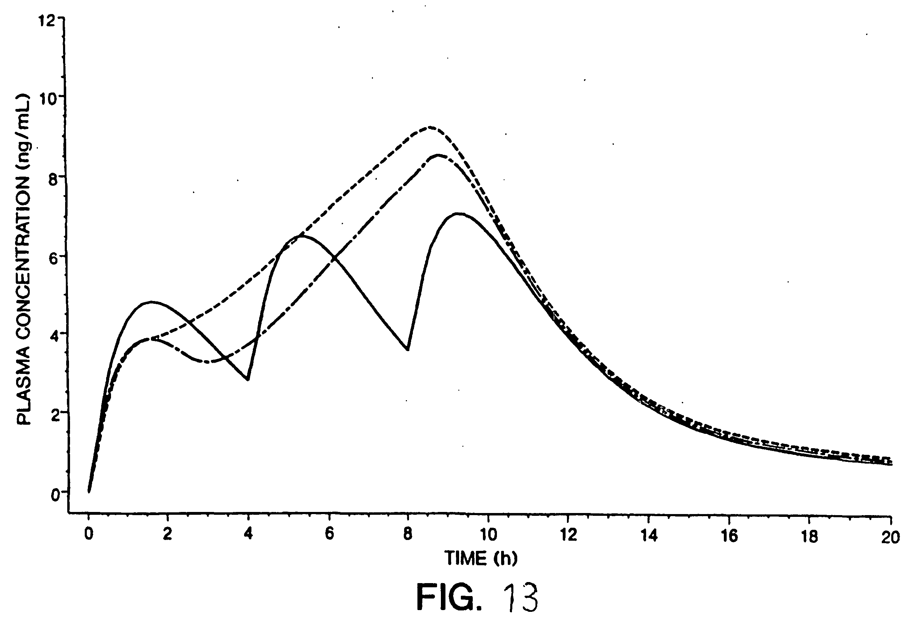

The accompanying drawing figures depict the therapeutic benefits obtained by the invention. Figure 6 illustrates a simulated plasma methylphenidlate concentration profile for three-times-a-day 30 mg dose (solid line), an ascend treatment of 36 mg (dash line), and an osmotic controlled 36 mg dose (dot-dash line). Figure 7 depicts the plasma methylphenidlate concentration as in Figure 6, except in Figure 7 the osmotic controlled dose is 38 mg. Figure 8 depicts the plasma methylphenidate concentration as in Figure 6, except in Figure 8, the osmotic controlled dose is 40 mg. Figure 9 illustrates: 30 mg delivered three times a day by the solid line, an ascend dose from a dosage form comprising 36 mg of drug once a day by the dash line, and a dosage form comprising an immediate 8 mg dose and a sustained 26 mg ascending dose illustrated by the dot-dash line. Figure 10 depicts a plasma methylphenidate concentration like Figure 9, except the dosage form represented by the dot-dash line comprises an instant-release dose of 9 mg of methylphenidate and an ascending dose of 24 mg of methylphenidate. Figure 11 is similar to the previous Figures except the dot-dash line depicts an instant release dose of 8 mg and an ascending dose of 25 mg of methylphenidate. Figure 12 is similar to the above Figures, except the dot dash line illustrates an immediate dose of methylphenidate of 8 mg followed by a sustained ascending dose of 25 mg of methylphenidate. Figure 13 is similar to the above Figures with the clinical conditions as set forth, except in this study the dot-dash lines illustrate an immediate dose of 8 mg of methylphenidate, followed by a controlled-ascending dose of 24 mg of methylphenidate.

## Claims

1. A pharmaceutical composition in a dosage form comprising a dose of a drug selected from methylphenidate, amphetamine, dextroamphetamine, methamphetamine, phenylisopropylamine, pemoline, and pharmaceutically acceptable salts thereof, in admixture with a pharmaceutically acceptable carrier, wherein said dosage form comprises drug releasing beads and is capable of delivering said drug in a continuously increasing release rate per hour for 4 to 8½ hours.

2. A pharmaceutical composition as claimed in claim 1 wherein said dosage form is capable of delivering said drug in a controlled increasing dose over an extended time to maintain the therapeutic effect of the drug, while concomitantly substantially avoiding the development of acute tolerance in a patient.

3. A pharmaceutical composition as claimed in claim 1 or claim 2 wherein said beads are coated with a release rate controlling polymer.

4. A pharmaceutical composition as claimed in any preceding claim comprising 1 mg to 500 mg of said drug.

5. A pharmaceutical composition as claimed in any preceding claim wherein said drug is released in a sustained and continuously ascending dose of 100 ng to 375 mg over 16 hours.

6. A pharmaceutical composition as claimed in any one of claims 1 to 5 for use in the treatment of Attention-Deficit Disorders.

7. A pharmaceutical composition as claimed in any one of claims 1 to 5 for use in the treatment of Attention-Deficit Hyperactivity Disorder.

8. Use of a drug selected from methylphenidate, amphetamine, dextroamphetamine, methamphetamine, phenylisopropylamine, pemoline, and pharmaceutically acceptable salts thereof in the manufacture of a medicament for use in a method of treating an Attention-Deficit Disorder in a patient, wherein said medicament comprises drug releasing beads and delivers said drug in a continuously increasing release rate per hour for 4 to 8½ hours .

9. Use as claimed in claim 8 wherein said medicament is orally administered to said patient and delivers 100 ng to 500 mg of said drug in a sustained and continuously ascending dose over 16 hours.

10. Use as claimed in claim 8 or claim 9 for lessening or completely compensating for tolerance to said drug in a patient having Attention-Deficit Disorder.

11. Use as claimed in any one of claims 8 to 10 for maintaining the therapeutic effect of said drug in an Attention-Deficit Disorder patient who acquires tolerance to said drug.

## Patentansprüche

1. Pharmazeutische Zubereitung in einer Dosierungsform, umfassend eine Dosis eines unter Methylphenidat, Amphetamin, Dextroamphetamin, Methamphetamin, Phenylisopropylamin, Pemolin und pharmazeutisch verträglichen Salzen davon ausgewählten Wirkstoffs im Gemisch mit einem pharmazeutisch verträglichen Träger, wobei die Dosierungsform Wirkstofffreisetzende Kügelchen umfasst und geeignet ist, den Wirkstoff über 4 bis 8 ½ Stunden in einer kontinuierlich zunehmenden Freisetzungsgeschwindigkeit pro Stunde abzugeben.

2. Pharmazeutische Zubereitung nach Anspruch 1, wobei die Dosierungsform geeignet ist, den Wirkstoff in einer gesteuert zunehmenden Dosis über eine längere Zeitspanne abzugeben, um die therapeutische Wirkung des Wirkstoffs zu erhalten und gleichzeitig die Ausbildung einer akuten Toleranz beim Patienten im Wesentlichen zu vermeiden.

**3.** Pharmazeutische Zubereitung nach Anspruch 1 oder 2, wobei die Kügelchen mit einem die Freisetzungsgeschwindigkeit steuernden Polymer überzogen sind.

**4.** Pharmazeutische Zubereitung nach einem der vorhergehenden Ansprüche, umfassend 1 mg bis 500 mg Wirkstoff.

**5.** Pharmazeutische Zubereitung nach einem der vorhergehenden Ansprüche, wobei der Wirkstoff über 16 Stunden in einer verzögerten und kontinuierlich ansteigenden Dosis von 100 ng bis 375 mg freigesetzt wird.

**6.** Pharmazeutische Zubereitung nach einem der Ansprüche 1 bis 5, zur Anwendung zur Behandlung von Aufmerksamkeitsdefizitstörungen.

**7.** Pharmazeutische Zubereitung nach einem der Ansprüche 1 bis 5, zur Anwendung zur Behandlung von Aufmerksamkeitsdefizit-Hyperaktivitätsstörungen.

**8.** Verwendung eines unter Methylphenidat, Amphetamin, Dextroamphetamin, Methamphetamin, Phenylisopropylamin, Pemolin und pharmazeutisch verträglichen Salzen davon ausgewählten Wirkstoffs zur Herstellung eines Arzneimittels zur Anwendung zur Behandlung von Aufmerksamkeitsdefizitstörung bei einem Patienten, wobei das Arzneimittel Wirkstofffreisetzende Kügelchen umfasst und den Wirkstoff über 4 bis 8 ½ Stunden in einer kontinuierlich zunehmenden Freisetzungsgeschwindigkeit pro Stunde abgibt.

**9.** Verwendung nach Anspruch 8, wobei das Arzneimittel dem Patienten oral verabreicht wird und 100 ng bis 500 mg des Wirkstoffs über 16 Stunden in einer verzögerten und kontinuierlich ansteigenden Dosis abgibt.

**10.** Verwendung nach Anspruch 8 oder 9, zur Verminderung oder vollständigen Kompensation der Toleranz gegenüber dem Wirkstoff bei einem Patienten mit Aufmerksamkeitsdefizitstörung.

**11.** Verwendung nach einem der Ansprüche 8 bis 10, zur Erhaltung der therapeutischen Wirkung des Wirkstoffs bei einem Patienten mit Aufmerksamkeitsdefizitstörung, der eine Toleranz gegenüber dem Wirkstoff erwirbt.

**Revendications**

**1.** Composition pharmaceutique sous une forme dosée comprenant une dose d'un médicament choisi parmi le phénidate de méthyle, l'amphétamine, la dextroamphétamine, la méthamphétamine, la phénylisopropylamine, la pémoline, et leurs sels acceptables pharmaceutiquement, en mélange avec un véhicule acceptable pharmaceutiquement, la forme dosée comprenant des billes de libération de médicament et étant apte à administrer le médicament à une vitesse horaire de libération augmentant continuellement pendant 4 à 8 heures et demie.

**2.** Composition pharmaceutique suivant la revendication 1, dans laquelle la forme dosée est apte à administrer le médicament, en une dose augmentant de manière réglée sur une durée prolongée pour maintenir l'effet thérapeutique du médicament tout en évitant sensiblement l'apparition d'une tolérance aiguë chez un patient.

**3.** Composition pharmaceutique suivant la revendication 1 ou la revendication 2, dans laquelle les billes sont enrobées d'un polymère réglant la vitesse de libération.

**4.** Composition pharmaceutique suivant l'une quelconque des revendications précédentes comprenant de 1 mg à 500 mg du médicament.

**5.** Composition pharmaceutique suivant l'une quelconque des revendications précédentes, dans laquelle le médicament est libéré en une dose maintenue et continuellement croissante de 100 mg à 375 mg en 16 heures.

**6.** Composition pharmaceutique suivant l'une quelconque des revendications 1 à 5 à utiliser dans le traitement de troubles de déficit de l'attention.

**7.** Composition pharmaceutique suivant l'une quelconque des revendications 1 à 5 à utiliser dans le traitement de troubles d'hyperactivité avec déficit de l'attention.

**8.** Utilisation d'un médicament choisi parmi le phénidate de méthyle, l'amphétamine, la dextroamphétamine, la méthamphétamine, la phénylisopropylamine, la pémoline, et leurs sels acceptables pharmaceutiquement, dans la fabrication d'un médicament à utiliser dans un procédé de traitement d'un trouble d'un déficit de l'attention chez un patient, le médicament comprenant des billes de libération du médicament et administrant le médicament à une vitesse horaire d'administration continuellement croissante pendant 4 à 8 heures et demie.

9. Utilisation suivant la revendication 8, dans laquelle le médicament est administré par voie orale au patient et administre de 100 mg à 500 mg du médicament en une dose qui se maintient et continuellement croissante en 16 heures.

10. Utilisation suivant la revendication 8 ou la revendication 9 pour amoindrir ou compenser complètement la tolérance au médicament chez un patient ayant un trouble de déficit de l'attention.

11. Utilisation suivant l'une quelconque des revendications 8 à 10 pour maintenir l'effet thérapeutique du médicament chez un patient souffrant d'un trouble de déficit de l'attention qui acquière de la tolérance au médicament.

FIG. 1

EP 0 932 388 B1

FIG. 2

FIG. 3

FIG. 4

FIG. 5

FIG. 6

FIG. 7

EP 0 932 388 B1

PLASMA CONCENTRATION (ng/mL)

TIME (h)

FIG. 8

FIG. 9

PLASMA CONCENTRATION (ng/mL)

TIME (h)

FIG. 10

FIG. 11

EP 0 932 388 B1

FIG. 12

FIG. 13